# EUROPÄISCHE PATENTSCHRIFT

(11) **EP 1 741 468 B1**
(45) Veröffentlichungstag und Bekanntmachung des Hinweises auf die Patenterteilung: **13.08.2008**
(21) Anmeldenummer: 06014097.7
(22) Anmeldetag: 07.07.2006
(51) Int. Cl.: A61N 5/06, H01S 5/227

(54) **Optische Strahlungsquelle für die Behandlung von lebendem biologischen Gewebe**
Optical radiation source for the treatment of living biologic tissue
Source du rayonnement optique pour le traitement de tissus vivants biologiques

(30) Priorität: 07.07.2005 DE 102005031906
(43) Veröffentlichungstag der Anmeldung: 10.01.2007
(73) Patentinhaber: Hauptmann, Gerhard, 81827 München (DE)
(72) Erfinder: Hauptmann, Gerhard, 81827 München (DE)

(56) Entgegenhaltungen:
- EP-A- 0 320 080
- WO-A-20/05107867
- US-A- 4 884 276
- US-A1- 2004 030 370
- US-A1- 2004 210 277
- US-A1- 2004 230 258
- US-A1- 2004 260 365

## Beschreibung

Die Erfindung betrifft eine optische Strahlungsquelle für die Behandlung von lebendem biologischen Gewebe mit einer Strahlung in einem Wellenlängenbereich zwischen 260 und 800 Nanometer.

Aus dem Patent DE 697 24 254 T2 ist eine Vorrichtung bekannt, die zur äußerlichen medizinischen Behandlung mit einer Licht emittierenden Vorrichtung vorgesehen ist. Die Strahlungsquelle ist dabei an einem Körper einer Person anzulegen oder in nächster Nähe des Körpers einer Person zu halten. Die Strahlungsquelle besteht aus einem Licht emittierenden Element, einer Licht emittierenden Diode oder korrespondierenden Elementen und ist so konstruiert, dass sie monochromes Licht einer ersten Wellenlänge emittiert und geeignet ist, monochromes Licht gleichzeitig nur einer Wellenlänge zu übertragen. Dabei wird das monochrome Licht über eine erste vorbestimmte Zeitdauer in einer ersten Stufe emittiert und danach selektiv monochromes Licht einer anderen Wellenlänge als der ersten Wellenlänge über eine zweite vorbestimmter Zeitdauer in einer möglichen zweiten Stufe emittiert. Die Antriebseinrichtung ist dabei so konstruiert, dass sie die Licht emittierende Vorrichtung veranlasst, das emittierte Licht gemäß einer vorbestimmten Pulsfrequenz oder Reihen von Pulsfrequenzen über bestimmte Zeitdauern gepulst abzugeben. Das pulsierende Licht wird mit einer Pulslänge emittiert, die innerhalb eines Intervalls von etwa 67 Prozent bis 88 Prozent der Zeit zwischen den entsprechenden Startflanken zweier gegenseitig sequenzieller Pulse liegt. Das Patent DE 697 24 254 T2 basiert auf der Erkenntnis, dass die medizinische Wirkung von der relativen Dauer eines Impulses innerhalb eines Intervalls bzw. einer Zeiteinheit abhängig ist. Dabei wird bei einer Impulslänge zwischen 67 Prozent bis 88 Prozent innerhalb eines Intervalls eine maximale medizinische Wirkung erreicht. Ein Hinweis auf bestimmte Frequenzen der Strahlungsquelle wird nicht gegeben.

Es ist weiterhin aus der Firmenschrift BTL-5000 Laser bekannt, einen Laser, beispielsweise einen Diodenlaser zur Gewebestimulation mit einer Frequenz, bis 10 kHz, zu modulieren.

Aus der Patentanmeldung, EP0320 080 A1 ist eine Vorrichtung bekannt, die alle Merkmale in Oberbegriff des Anspruchs 1 umfasst.

Es ist Aufgabe der vorliegenden Erfindung, die Behandlung von lebendem biologischen Gewebe mittels einer optischen Strahlungsquelle spezifisch auf den jeweiligen Organismus, oder eines seiner Organe abstimmen zu können und somit die Wirkung der Bestrahlung zu verbessern.

In der vorliegenden Erfindung gilt es bei der Behandlung von lebendem biologischen Gewebe, den Organismus nicht universell und unisono zu therapieren, sondern Teilbereiche eines Körpers bzw. Organe selektiv zu therapieren. Dies gelingt durch eine optische Strahlungsquelle mit den Merkmalen des Anspruchs 1. Die Wellenlänge der Strahlung ist dabei so gewählt, dass diese auf das zu therapierenden Gewebe wirkt. Die selektive Resonanz und somit selektive Therapie einzelner Organe wird durch die Kombination der folgenden physikalischen Parameter ermöglicht.

Der Wellenlängenbereich liegt in dem von F.A. Popp (Popp F.A., Biophotonen, Verlag für Medizin, Dr. Ewald Fischer, Heidelberg 1976) definierten Bereich der Biokommunikation zwischen 260 und 800 Nanometer. Durch umfangreiche eigene Untersuchungen konnte eine wirksame Frequenzmodulation der Laserstrahlung zwischen ein 200 kHz und 20 MHz ermittelt werden. In diesem Frequenzbereich wird in bezug auf den qualitativen und zeitlichen Verlauf des Heilungsprozesses die Wirksamkeit der Laserstrahlung deutlich verstärkt. Etwa 95 Prozent der ermittelten Frequenzen liegen in einem Bereich zwischen 300 kHz und 10 MHz. Die individuellen Frequenzen sind innerhalb der oben genannten Frequenzbereiche kontinuierlich einstellbar. Die Frequenz wird mit einem konstanten Puls-Pausen Verhältnis von idealerweise 50:50 einfach moduliert. Eine einfache Modulation besteht, wenn die einzelnen Impulse der Basis- oder Grundmodulation nicht zusätzlich moduliert sind. Bei Änderung der Modulationsfrequenz ändert sich bei einem konstanten Puls-Pausenverhältnis die abgegebene Leistung nicht. Zur optimalen Übertragung der optischen Strahlung an das für die Behandlung vorgesehene lebende biologische Gewebe ist eine Leistungsdichte zwischen 0,1 und 10 W/cm² erforderlich. Idealerweise ist die optische Strahlung eine kohärente Laserstrahlung, die von einem Halbleiterlaser emittiert wird. Es ist bekannt, dass Wellenlängen im Bereich um 635 nm von der DNS besonders gut geleitet bzw. transportiert werden. Eine Wellenlänge von 635 Nanometer hat sich dabei als besonders günstig für die Übertragung an das lebende biologische Gewebe und somit für den Behandlungserfolg herausgestellt. Zur Erzeugung dieser Strahlung eignen sich die verfügbaren Laserstrahlquellen wie z.B. Gas- oder Halbleiterlaser. Die optische Strahlung kann entweder direkt von dem strahlerzeugenden Element auf das zu behandelnde Gewebe gerichtet, oder mittels Lichtleiter übertragen werden. Diese Art der Übertragung reduziert die Erzeugung einer unerwünschten therapeutischen Wärme auf ein Minimum. Für den Behandlungserfolg ist eine Einstellgenauigkeit der Modulationsfrequenz und eine Frequenzstabilität von besser als 0,1 Prozent besonders vorteilhaft. Dies wird nach dem technischen Stand entsprechend durch quarzgeregelte Funktionsgeneratoren erreicht. Zur Ermittelung der für die Behandlung erforderlichen Modulationsfrequenz wird der, in der Schulmedizin anerkannte, kinesiologische Muskeltest (Klinghardt D. Dr. med., Lehrbuch der Psycho-Kinesiologie, Bauer Verlag, 1999, S. 110-112) zu Grunde gelegt. Auf Basis dieses Muskeltests können die Modulationsfrequenzen mit der erforderlichen Genauigkeit von 0,1 Prozent und besser bestimmt werden.

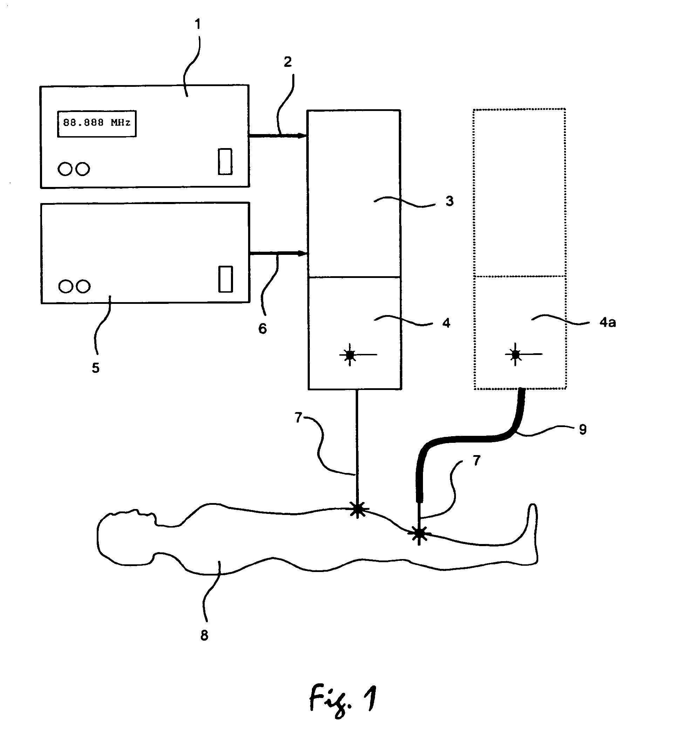

Der besondere Vorteil der Erfindung ist darin zu sehen, dass das Therapieverfahren zum Beispiel individuell auf den einzelnen Patienten und dessen individuelles Krankheitsbild abgestimmt wird. Ein Ausführungsbeispiel gemäß der Erfindung ist in der Fig. 1 dargestellt und wird im Folgenden beschrieben:
Fig. 1 zeigt schematisch vereinfacht das Lasertherapiesystem mit folgenden Teilkomponenten. Ein Frequenzgenerator 1 dient zur Modulation der Laserstrahlung 7 und liefert ein digitales TTL Signal über ein hochfrequenzgeeignetes Kabel 2 an das Stromregelmodul 3 der Laserdiode 4. Zur Energieversorgung der Laserdiode dient das Spannungsversorgungsmodul 5, das über ein Kabel 6 ebenfalls an das Stromregelmodul 3 der Laserdiode 4 angeschlossen ist. Das Stromregelmodul 3 steuert nun die Laserdiode 4 in deren Intensität, die somit wiederum ein digital moduliertes kohärentes Laserlicht 7 emittiert. Der Bereich der Frequenzmodulation bzw. die Modulationsfrequenz wird vor Behandlung mittels kinesiologischen Muskeltest (siehe Literaturangabe) individuell ermittelt und liegt bei 95 Prozent der Patienten im Bereich von 0,3 MHz bis 10 MHz. Für eine kostengünstige Realisierung ist daher dieser Frequenzbereich ausreichend. Die Erfahrung zeigt, dass die Modulationsfrequenz vor jeder Behandlung überprüft werden sollte, da diese sich im Verlauf der Therapie entsprechend der Gesundung eines Patienten 8 verändern kann. Die Intensität der Laserstrahlung 7 wird zwischen 0% und 100% moduliert. Aus technischen Gründen, die durch die Technologie von Laserdioden vorgegeben sind, ist es vorteilhaft, die Intensität nicht auf null zu modulieren, sondern eine geringe Restintensität der Laserstrahlung beizubehalten. Diese Art der Modulation verbessert zum einem die Lebensdauer von Laserdioden und zum anderen wird die maximal mögliche Modulationsfrequenz erhöht. Die Restintensität der Laserstrahlung 7 liegt in einem Bereich, der therapeutisch nicht mehr wirksam ist. Die derart modulierte Laserstrahlung 7 mit einer Wellenlänge von 635 nm wird mit einer Leistungsdichte von ca. 1 W/cm² auf das zu behandelnde lebende biologische Gewebe 8 gerichtet. In dem gewählten Ausführungsbeispiel wird die Laserstrahlung 7 als kollimierter Parallelstrahl übertragen. Diese Übertragungsart bietet den Vorteil einer vom Behandlungsabstand weitgehend unabhängigen Leistungsdichte der Laserstrahlung. Die Laserstrahlung wird auf das biologische Gewebe appliziert. Alternativ wie in der Fig. 1 auf der rechten Seite dargestellt ist, kann die Übertragung der Laserstrahlung mit optischen Übertragungselementen, wie zum Beispiel einem Lichtleiter 9, erfolgen und als Strahlquelle ein Laserdiodenarray, bestehend aus mehreren Einzeldioden 4a, verwendet werden.

Alternativ zum Frequenzgenerator 1 kann die Frequenzmodulation auch durch ein Chopperrad erfolgen. Dabei wird die Laserstrahlung 7 kontinuierlich emittiert und mittels Chopperrad moduliert. Eine entsprechende Drehzahlgenauigkeit und Drehzahlstabilität des Chopperrades sind dabei einzuhalten.

## Patentansprüche

1. Optische Strahlungsquelle für die athermische Behandlung von lebendem biologischen Gewebe (8) mit einer monochromatischen Strahlung (7) aus einem Wellenlängenbereich zwischen 260 und 800 Nanometer, wobei die Strahlung in einem Frequenzbereich zwischen 200 kHz und 20 MHz einfach moduliert ist, **dadurch gekennzeichnet, dass** die Modulationsfrequenz quarzgeregelt erzeugt ist und sowohl eine Einstellgenauigkeit als auch eine Frequenzstabilität von besser als 0,1 % aufweist, wobei die Modulationsfrequenz durch einen kinesiologischen Muskeltest bestimmt ist.

2. Optische Strahlungsquelle nach Anspruch 1, **dadurch gekennzeichnet, dass** die Modulationsfrequenz in einem Frequenzbereich zwischen 300 kHz und 10 MHz liegt.

3. Optische Strahlungsquelle nach Anspruch 1 oder 2, **dadurch gekennzeichnet, dass** die Leistungsdichte der Strahlung (7) zwischen 0,1 und 10 W/cm² beträgt.

4. Optische Strahlungsquelle nach einem der Ansprüche 1 bis 3, **gekennzeichnet durch** mindestens einen Halbleiterlaser (4).

5. Optische Strahlungsquelle nach einem der Ansprüche 1 bis 4 **dadurch gekennzeichnet, dass** die Wellenlänge der Strahlung (7) 635 Nanometer beträgt.

6. Optische Strahlungsquelle nach einem der Ansprüche 1 bis 5, **dadurch gekennzeichnet, dass** die modulierte optische Strahlung (7) kollimiert auf das biologische Gewebe (8) appliziert ist.

7. Optische Strahlungsquelle nach einem der Ansprüche 1 bis 6, **dadurch gekennzeichnet, dass** die modulierte optische Strahlung (7) über einen Lichtleiter (9) auf das biologische Gewebe appliziert ist.

## Claims

1. Optical radiation source for the non thermal treatment of living biologic tissue (8) with a monochromatic radiation (7) with a wavelength range between 260 and 800 nanometres, the radiation in a frequency range between 200 kHz and 20 MHz being single modulated, **characterised in that** the radiation source (7) is quartz-crystal controlled generated and comprises a setting accuracy and frequency stability of better than 0.1 %, where the modulation frequency is determined by means of a kinesiologic muscle test.

2. Optical radiation source according to claim 1, **characterised in that** the modulation frequency lies in a frequency range from 300 kHz to 10 MHz.

3. Optical radiation source according to claim 1 or 2, **characterised in that** the power density of the radiation (7) is between 0.1 and 10 W/cm².

4. Optical radiation source according to any of claims 1 to 3, **characterised by** at least one semiconductor laser.

5. Optical radiation source according to any of claims 1 to 4, **characterised in that** the wavelength of the radiation (7) is 635 nanometres.

6. Optical radiation source according to any of claims 1 to 5, **characterised in that that** modulated optical radiation (7) is applied in collimated form to the biologic tissue (8).

7. Optical radiation source according to any of claims 1 to 6, **characterised in that** the modulated optical radiation (7) is applied by means of a light guide to the biologic tissue.

## Revendications

1. Source du rayonnement optique pour le non thermique traitement de tissus biologiques vivants (8) avec un rayonnement monochromatique (7) d'un gamme de longueur d'onde entre 260 et 800 nanomètres, le rayonnement (7) étant seul modulé avec une gamme de fréquence entre 200 kHz et 20 MHz **caractérisé en ce que** la fréquence de modulation est commandé par cristal et a une précision de réglage et une stabilité de fréquence qui sont les deux meilleur que 0,1% dont la fréquence de modulation est déterminée par une test de muscle kinsesiologique.

2. Source du rayonnement optique selon la revendication 1, **caractérisée en ce que** la fréquence de modulation est dans une gamme de fréquence de 300 kilohertz a 10 mégahertz.

3. Source du rayonnement optique selon la revendication 1 ou 2, **caractérisé en ce que** la densité de puissance du rayonnement (7) est entre 0.1 et 10 W/cm².

4. Source du rayonnement optique selon la revendication 1 à 3. **caractérisé par** au moins un laser de semi-conducteur.

5. Source du rayonnement optique selon ta revendication 1 à 4, **caractérisé en ce que** la longueur d'onde du rayonnement (7) est de 635 nanomètres.

6. Source du rayonnement optique selon la revendication 1 à 5, **caractérisé en ce que** le rayonnement optique modulé (7) est appliqué sous la forme collimatée au tissu biologique (8).

7. Source du rayonnement optique selon la revendication 1 à 6, **caractérisé en ce que** le rayonnement optique modulé (7) est appliquée au moyen d'un guide de lumière au tissu biologique.
